# EUROPEAN PATENT SPECIFICATION

(11) **EP 2 545 531 B1**
(45) Date of publication and mention of the grant of the patent: **13.06.2018**
(21) Application number: 11753828.0
(22) Date of filing: 03.03.2011
(51) Int. Cl.: G06T 15/00, G06T 7/00

(54) **SYSTEM FOR SKIN TREATMENT ANALYSIS USING SPECTRAL IMAGE DATA TO GENERATE 3D RGB MODEL**
SYSTEM ZUR ANALYSE EINER HAUTBEHANDLUNG MIT SPEKTRALBILDDATEN ZUR ERZEUGUNG EINES 3D-RGB-MODELLS
SYSTÈME D'ANALYSE DE TRAITEMENT DE LA PEAU UTILISANT DES DONNÉES D'IMAGES SPECTRALES AFIN DE GÉNÉRER UN MODÈLE RGB 3D

(30) Priority: 10.03.2010 US 312559 P
(43) Date of publication of application: 16.01.2013
(73) Proprietor: ELC Management LLC, New York, NY 10153 (US)
(72) Inventor: CUMMINS, Phillip, Livingston New Jersey 07039 (US); VANDEROVER, Garrett William, Bellerose New York 11426 (US); FTHENAKIS, Christina G., Dix Hills New York 11746 (US); JORGENSEN, Lise W., Trumbull Connecticut 06611 (US)
(74) Representative: Hirsch & Associés
(86) International application number: PCT/US2011/027057
(87) International publication number: WO 2011/112422

(56) References cited:
- EP-A1- 1 880 657
- WO-A1-2009/139879
- WO-A2-01/29769
- US-A1- 2007 110 305
- US-A1- 2009 303 342
- US-A1- 2009 306 484
- NORIMICHI TSUMURA ET AL: "Image-based skin color and texture analysis/synthesis by extracting hemoglobin and melanin information in the skin", ACM TRANSACTIONS ON GRAPHICS (TOG), ACM, US, vol. 22, no. 3, 1 July 2003 (2003-07-01), pages 770-779, XP058249586, ISSN: 0730-0301, DOI: 10.1145/882262.882344

## Description

### FIELD OF THE INVENTION

This invention relates to improved systems for analyzing and tracking skin conditions of a subject via photographs of the subject. In particular, it relates to an improved system and method for analyzing and tracking skin conditions via spectral and/or RGB format photographs of a subject, and simulating and/or tracking results of treatment of such skin conditions. The invention further relates to displaying such changing and treated conditions in an RGB image format on a three dimensional virtual model to facilitate research and consumer communication.

### BACKROUND OF THE INVENTION

The use of spectral imaging for tissue analysis and diagnosis is known as disclosed, for example, in U.S. Pat. No. 5,016,173 to Kenet et al., incorporated herein in its entirety by reference. Kenet et al. discloses an apparatus and method for in vivo monitoring of visually accessible surfaces of the body, including subsurface morphology. Kenet et al. teaches the combination of multiple digital photography techniques, including multispectral and multiview and/or multiresolution photographic methods to characterize and classify surface structure components and their temporal-spacial distributions.

A problem with the prior art is that it relies primarily on spectral imaging equipment for data capture, analysis and display. This is because spectral imaging allows levels of detail and analysis that are not possible within the visible light limitations of RGB format imaging equipment and photographs.

Unfortunately, spectral imaging equipment is relatively complex in structure and use, and of limited availability, e.g., it is better suited for laboratory or clinical use by trained technicians. Accordingly, until spectral imaging equipment becomes more widely available in simpler forms, the apparatus taught by prior art references such as Kenet may not be practical for wider distribution and use, such as, for example, by consumers in a retail environment or by users in a home environment.

Similarly, by its nature, spectral image data is difficult for the untrained eye to understand, view and/or analyze. Spectral image data is typically displayed in an abstract-art-like image with color separation that is confusing at best to the untrained eye. Accordingly, even if spectral image equipment becomes more widely available in more user friendly forms, data and images produced from the equipment is unlikely to be useful to the general public at large. Accordingly, the prior art systems are not useful on a broader scale, such as in a retail environment as a marketing tool.

Accordingly, there is a need for a system that is simple but effective, i.e., that permits use in non-laboratory or non-clinical circumstances, using widely available consumer oriented image equipment such as, for example, conventional digital cameras or the digital cameras that are commonly found in telephones, computers, personal digital assistants (PDA's) or other consumer electronics devices. There is further a need for a system that produces images and data that is easy to understand, analyze and view, even for the untrained eye.

WO2009139879 A1 relates to systems and methods for medical imaging;

WO0129769 A2 relates to method and apparatus for aligning and comparing images of the face and body from different images.

### SUMMARY OF THE INVENTION

The present invention provides a method for tracking and analyzing changing skin conditions and displaying such conditions in an RGB image format on a three-dimensional virtual model to facilitate research and consumer communication as set out in the appended claims 1 to 5 and the corresponding system set out in appended claim 6.

The method involves building a catalog, library or database of skin conditions in the form of datasets taken from spectral images that include the skin conditions of interest. For each spectral image dataset identifying a skin condition of interest a corresponding RGB dataset is calculated and compiled in a database. The database of calculated RGB datasets can then be used to diagnose skin conditions of subjects by, for example, analyzing RGB or spectral photographs of the subject. The spectral and/or RGB data sets can also be used to predict the effects of proposed treatments and the resulting altered skin condition can be displayed in RGB images that are readily comprehended by a larger audience than is presently possible solely with spectral images.

### DESCRIPTION OF DRAWINGS

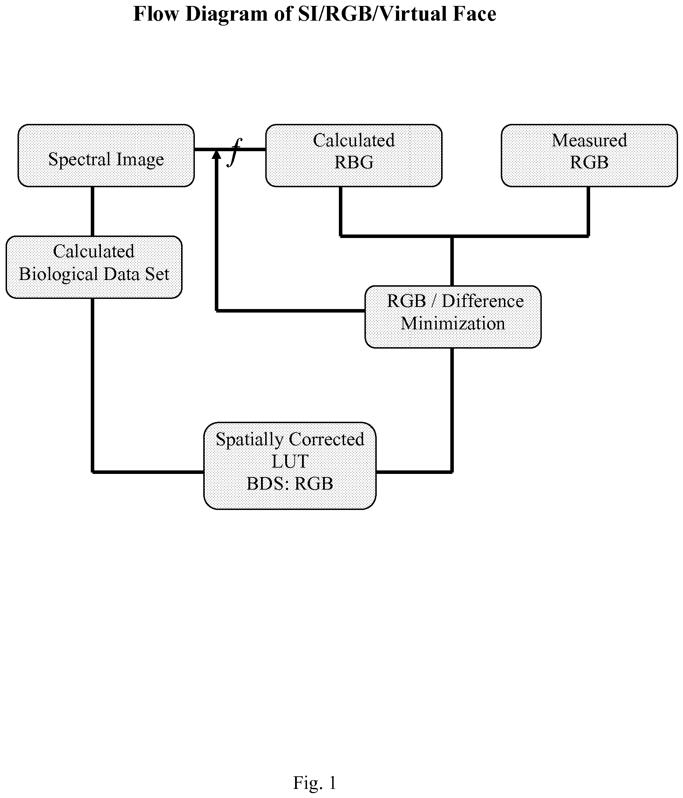

Fig. 1 is a flow diagram showing how an RGB dataset and a spectral image dataset are used to create a virtual look up table (LUT).

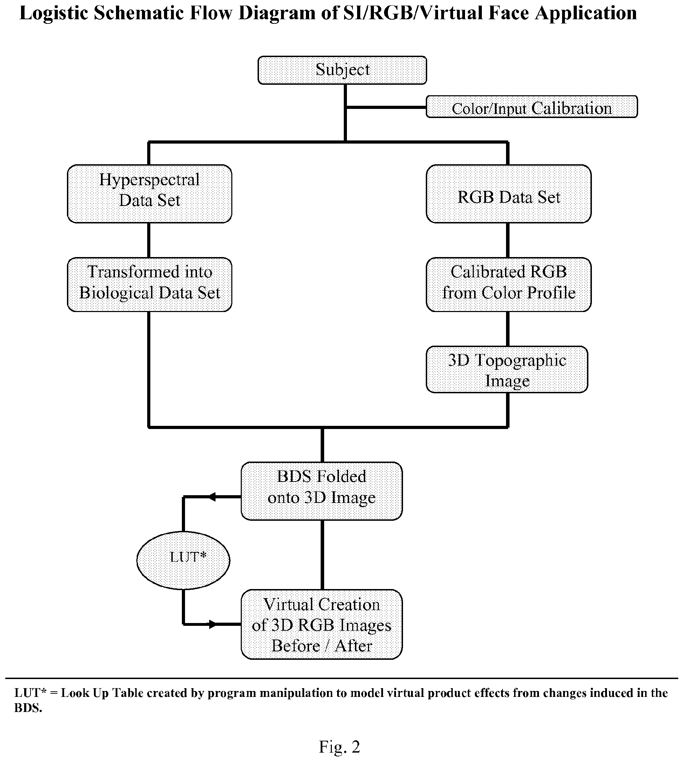

Fig. 2 is a flow diagram showing how captured and compiled information is used to analyze the skin conditions of an individual subject by capturing either spectral or RGB two dimensional photographs ("subject spectral images" or subject RGB images") of the individual subject and comparing datasets taken from the photographs to the reference datasets in the database(s) (LUT).

### DETAILED DESCRIPTION

Data bases are compiled using facial images captured from a large number of human subjects from a spectral camera, and a digital camera. The images are linked to specific skin conditions and are obtained under standard lighting conditions and internally calibrated.

More specifically, a plurality of two-dimensional digital spectral images ("spectral image" or "spectral images") of human skin are captured from a variety of human subjects and stored in a database. Each spectral image defines a target area of skin ("target" or "targets"). A corresponding plurality of two-dimensional digital RGB (red, green, blue) color model images ("RGB image" or "RGB images") are captured and stored in the same or a second database. Each of the RGB images corresponds at least in part to at least one of the spectral images defining a target. At least some of the plurality of spectral images is analyzed to identify within the respective spectral image one or more spectral image datasets. As used herein, "spectral image dataset" or "spectral image datasets" is the minimum amount of spectral image digital data required to uniquely define a condition of the skin ("skin condition"), as, for example, associated with a particular skin type, blood or melanin level, oxygen saturation, percent hemoglobin, deral scattering effect, percent water or moisture content, etc. The defined skin condition may be a skin condition not needing treatment or correction (for discussion purposes referred to herein as 'normal' skin conditions), or the defined skin condition may be a treatable or correctable skin condition such as, for example, dry, oily, cracked, and other treatable, correctable skin conditions. In any case, each spectral image dataset defines at least one skin condition.

Each element within each image within each database is recorded and indexed for pixel coordinates on the image, RGB value of the pixel or spectral content of the pixel, and type of skin condition at that pixel. Thus each skin condition is "mapped" in the respective image.

More specifically, each spectral image dataset is mapped to a location within the respective spectral image. The mapped location is referred to herein as the "spectral location", i.e., the pixel coordinate location within a spectral image for a spectral image dataset. In an RGB image corresponding to the respective spectral image, a location is mapped that corresponds to each spectral location. The location in the RGB image is referred to herein as the "RGB location", i.e., the pixel coordinate location within an RGB image that corresponds to a spectral location in a respective spectral image. For each spectral location, an RGB dataset is determined using standard functions (e.g., as disclosed in Berns, Roy. Billmeyer and Saltzman's Principles of Color Technology. Third Edition. New York, NY: John Wiley & Sons, 2000. 201-203. Print., incorporated herein by reference in its entirety). As used herein, "RGB dataset" or "RGB datasets" refers to the minimum amount of digital RGB data required to uniquely identify an RGB color profile associated with that respective location. In this way the spectral image dataset is effectively correlated to an RGB dataset that corresponds to at least one known skin condition defined by said spectral image dataset.

In this way, an RGB dataset is created pixel by pixel from each spectral dataset by passing the spectral data through a conversion function with the area under each resulting curve being summed to provide the RGB dataset. The conversion function is optimized from the minimization of the differences between the measured RGB values in RGB and those values calculated from the transformation RGB of the spectral dataset.

In this way a virtual look up table (LUT) between the RGB dataset and the spectral image dataset is established which is representative across all spectral image datasets within the database. It is expected that this method of averaging will be sufficient as representation within a given skin color type is a small variation in color space. It can however be extended to averaging in such a way as to represent the continuum of skin colors types experienced.

In this way, the different skin conditions are cataloged in spectral datasets and corresponding to determinable 'reference' RGB datasets. The captured spectral images and corresponding captured RGB images are compiled in one or more databases in a computer storage medium, along with the spectral image datasets representing skin conditions, the spectral locations, the RGB locations and the reference RGB datasets. The reference RGB datasets may be considered 'nonoptimized' as they contain relatively less precise data both quantitatively and qualitatively when compared to spectral datasets for the same pixel coordinates. However, they are sufficiently optimized for subsequent use in the analysis of subject RGB image data captured by widely available consumer oriented image equipment such as, for example, conventional digital cameras or the digital cameras that are commonly found in telephones, computers, personal digital assistants (PDA's) or other consumer electronics devices.

The captured and compiled information is used to analyze the skin conditions of an individual subject by capturing either spectral or RGB two dimensional photographs ("subject spectral images" or subject RGB images") of the individual subject and comparing datasets taken from the photographs to the reference datasets in the database(s). The resulting analysis can be used to recommend treatments for various skin conditions. The captured two dimensional images of the individual subject are also assembled in a composite on a three-dimensional frame to create an interactive, rotatable, virtual three-dimensional RGB image or model displaying the identified skin conditions, both 'normal' and treatable, in the locations on model corresponding to actual locations on subject. The database information is further used to generate on the three-dimensional RGB image an alteration of the displayed skin conditions resulting from application of treatments.

An advantage of the present invention is that RGB images alone can be used as the basis for the analysis of a subject. As RGB photography equipment is ubiquitous, cheaply and readily available, the images used for analysis can be taken almost anywhere, e.g., in a retail setting at counter, at a solon, at home, or on-the-go with a camera in a personal digital assistant or cell phone. There is no need for expensive, specialized spectral imaging cameras in laboratory settings.

Another advantage of the present invention is that it uses real time data to display resulting images that are "realistic" or "actual" projections of results from a treatment - i.e., virtual renderings of what the results will be. This is in contrast to current systems that merely estimate the results without underlying actual data.

Another advantage of the present invention is that either RGB or spectral images of a subject can be used as the basis for the analysis of the subject.

Once the database and LUT of spectral data sets correlated to RGB data sets is established, the system requires three basic steps: 1) take an RGB picture, 2) normalize (standardize) the RGB image via standard ICC profiling software to calibrate the color, intensity, etc. across various devices, and 3) compare the normalized data sets of the RGB image to the LUT to determine corresponding spectral image data sets, and in turn, the skin conditions associated with the spectral image datasets.

## Claims

1. A method for tracking and analyzing changing skin conditions and displaying such conditions in an RGB image format on a three-dimensional virtual model to facilitate research and consumer communication, the method comprising the steps of:
- capturing a plurality of digital spectral images of human skin, each spectral image defining a target;
- capturing a plurality of digital RGB images, each RGB image corresponding at least in part to at least one of the spectral images defining a target;
- analyzing at least some of the plurality of spectral images to identify within the respective spectral image one or more spectral image datasets, each spectral image dataset defining at least one skin condition;
- mapping within the respective spectral image one or more spectral locations for each of the one or more spectral image datasets;
- mapping within each RGB image corresponding to the respective spectral image one or more RGB locations corresponding to the respective one or more spectral locations of the each of the one or more spectral image datasets; and
- calibrating an RGB dataset corresponding to the spectral image dataset associated with that respective spectral location and mapping it to the RGB location such that the at least one known skin condition defined by said spectral image dataset can be reproduced in RGB format via the corresponding RGB image dataset;
**characterized in that** the method further comprises the steps of:
- compiling a database of said plurality of spectral images, said spectral image datasets, said corresponding skin conditions, said spectral locations, said plurality of RGB images, said RGB datasets and said RGB locations;
- capturing one or more digital RGB images, each digital image capturing an area of skin of a subject;
- analyzing each digital RGB image to locate any predetermined RGB datasets from the database;
- mapping the locations of the RGB datasets within each RGB image; and
- overlaying the RGB image onto a three-dimensional virtual frame to create a virtual three-dimensional model of the subject showing realistic skin conditions in locations on models corresponding to actual locations on subject.

2. The method of claim 1, wherein at least some of the plurality of digital spectral images are two-dimensional images.

3. The method of claim 1, wherein at least some of the plurality of digital spectral images are three-dimensional images.

4. The method of claim 1, wherein at least some of the plurality of digital RGB images are two-dimensional images.

5. The method of claim 1, wherein at least some of the plurality of digital RGB images are three-dimensional images

6. A system for use in analyzing human skin condition using a two-dimensional spectral image, the system comprising a computer including a processor and a memory for storing instructions for the processor to perform the method according to any one of claims 1 to 5.

## Patentansprüche

1. Ein Verfahren zur Nachverfolgung und Analyse von Hautzustandsveränderungen und zur Darstellung solcher Zustände in einem RGB-Bildformat auf einem dreidimensionalen virtuellen Modell, für die Vereinfachung von Forschung und Verbraucherkommunikation, wobei das Verfahren folgende Schritte umfasst:
- das Aufnehmen einer Vielzahl von digitalen Spektralbildern der menschlichen Haut, wobei jedes Spektralbild ein Zielobjekt definiert;
- das Aufnehmen einer Vielzahl von digitalen RGB-Bildern, wobei jedes RGB-Bild, mindestens teilweise, mindestens einem Spektralbild entspricht, das ein Zielobjekt definiert;
- das Analysieren mindestens einiger aus der Vielzahl von Spektralbildern, um innerhalb des entsprechenden Spektralbilds einen oder mehrere Spekralbilddatensätze zu ermitteln, wobei jeder Spekralbilddatensatz mindestens einen Hautzustand definiert;
- das Mappen, innerhalb des entsprechenden Spektralbilds, einer oder mehrerer Spektralstellen für jeden des einen oder der mehreren Spekralbilddatensätze;
- das Mappen, innerhalb von jedem, dem jeweiligen Spektralbild entsprechenden RGB-Bild einer oder mehrerer RGB-Stellen, die den jeweiligen einen oder mehreren Spektralstellen von jedem der einen oder der mehreren Spekralbilddatensätzen entsprechen, und;
- das Kalibrieren eines RGB-Datensatzes der dem Spekralbilddatensatz entspricht, der mit der jeweiligen Spektralstelle assoziert ist, und dessen Mappen zur RGB-Stelle, so dass der mindestens eine, vom Spekralbilddatensatz definierte, bekannte Hautzustand im RGB-Format über den entsprechenden RGB-Bilddatensatz reproduziert werden kann;
**dadurch gekennzeichnet dass** das Verfahren ferner folgende Schritte umfasst:
- das Erstellen einer Datenbank aus der Vielzahl der Spektralbilder, der Spekralbilddatensätze, der entsprechenden Hautzustände, der Spektralstellen, der Vielzahl der RGB-Bilder, der RGB-Datensätze und der RGB-Stellen;
- das Aufnehmen eines oder mehrerer digitaler RGB-Bilder, wobei jede digitale Bildaufnahme einen Hautbereich eines Subjekts aufnimmt;
- das Analysieren jedes digitalen RGB-Bilds um jegliche vorbestimmte RGB-Datensätze aus der Datenbank zu lokalisieren;
- das Mappen der Stellen der RGB-Datensätze zu jedem RGB-Bild; und
- das Überlagern des RGB-Bilds auf einen dreidimensionalen virtuellen Frame zum Erstellen eines virtuellen, dreidimensionalen Modells des Subjekts, welches realistische Hautzustände an Stellen von Modellen zeigt, die den aktuellen Stellen am Subjekt entsprechen.

2. Das Verfahren nach Anspruch 1, wobei mindestens einige aus der Vielzahl von digitalen Spektralbildern zweidimensionale Bilder sind.

3. Das Verfahren nach Anspruch 1, wobei mindestens einige aus der Vielzahl von digitalen Spektralbildern dreidimensionale Bilder sind.

4. Das Verfahren nach Anspruch 1, wobei mindestens einige aus der Vielzahl von digitalen RGB-Bildern zweidimensionale Bilder sind.

5. Das Verfahren nach Anspruch 1, wobei mindestens einige aus der Vielzahl von digitalen RGB-Bildern dreidimensionale Bilder sind.

6. Ein System zur Anwendung bei der Analyse für die Behandlung von menschlicher Haut unter der Verwendung eines zweidimensionalen Spektralbilds, wobei das System einen Computer umfasst, der einen Prozessor und einen Speicher zum Speichern von Befehlen enthält, so dass der Prozessor das Verfahren nach einem der Ansprüche 1 bis 5 durchführt.

## Revendications

1. Procédé de suivi et d'analyse de changements d'états cutanés et d'affichage de ces états sous la forme d'une image RGB sur un modèle virtuel en trois dimensions afin de faciliter la recherche et la communication avec un consommateur, le procédé comprenant les étapes suivantes :
- la capture d'une pluralité d'images spectrales numériques de la peau humaine, chaque image spectrale définissant une cible ;
- la capture d'une pluralité d'images RGB numériques, chaque image RGB correspondant au moins en partie à au moins l'une des images spectrales définissant une cible ;
- l'analyse d'au moins certaines de la pluralité d'images spectrales afin d'identifier, sur l'image spectrale respective, un ou plusieurs ensembles de données d'image spectrale, chaque ensemble de données d'image spectrale définissant au moins un état cutané ;
- le mappage, sur l'image spectrale respective, d'un ou plusieurs emplacements spectraux pour chacun du ou des ensembles de données d'image spectrale ;
- le mappage, sur chaque image RGB qui correspond à l'image spectrale respective, d'un ou plusieurs emplacements RGB qui correspondent au ou aux emplacements spectraux respectifs de chacun du ou des ensembles de données d'image spectrale ; et
- le calibrage d'un ensemble de données RGB qui correspond à l'ensemble de données d'image spectrale associé à cet emplacement spectral respectif, et son mappage par rapport à l'ensemble RGB de sorte que le au moins un état cutané connu défini par ledit ensemble de données d'image spectrale puisse être reproduit au format RGB via l'ensemble de données d'image RGB correspondant ;
**caractérisé en ce que** le procédé comprend en outre les étapes suivantes :
- la compilation d'une base de données de ladite pluralité d'images spectrales, desdits ensembles de données d'image spectrale, desdits états cutanés correspondants, desdits emplacements spectraux, de ladite pluralité d'images RGB, desdits ensembles de données RGB et desdits emplacements RGB ;
- la capture d'une ou plusieurs images RGB numériques, chaque image numérique capturant une zone de la peau d'un sujet ;
- l'analyse de chaque image RGB numérique afin de localiser les ensembles de données RGB prédéterminés issus de la base de données ;
- le mappage des emplacements des ensembles de données RGB sur chaque image RGB ; et
- la superposition de l'image RGB sur un cadre virtuel en trois dimensions afin de créer un modèle virtuel en trois dimensions du sujet qui indique des états cutanés réalistes à des emplacements du modèle qui correspondent aux emplacements réels sur le sujet.

2. Procédé selon la revendication 1, dans lequel au moins certaines de la pluralité d'images spectrales numériques sont des images en deux dimensions.

3. Procédé selon la revendication 1, dans lequel au moins certaines de la pluralité d'images spectrales numériques sont des images en trois dimensions.

4. Procédé selon la revendication 1, dans lequel au moins certaines de la pluralité d'images RGB numériques sont des images en deux dimensions.

5. Procédé selon la revendication 1, dans lequel au moins certaines de la pluralité d'images RGB numériques sont des images en trois dimensions.

6. Système destiné à être utilisé pour analyser un état cutané humain à l'aide d'une image spectrale en deux dimensions, le système comprenant un ordinateur qui comprend un processeur et une mémoire destinée à stocker des instructions pour permettre au processeur d'exécuter le procédé selon l'une quelconque des revendications 1 à 5.
